## Europäisches Patentamt

(19) **European Patent Office**

**Office européen des brevets**

(11) Veröffentlichungsnummer : **0 282 551 B2**

(12) **NEUE EUROPÄISCHE PATENTSCHRIFT**

(45) Veröffentlichungstag der neuen Patentschrift :
**20.07.94 Patentblatt 94/29**

(51) Int. Cl.⁵ : **A61K 7/13,** D06P 1/32,
D06P 3/08

(21) Anmeldenummer : **87906173.7**

(22) Anmeldetag : **19.08.87**

(86) Internationale Anmeldenummer :
**PCT/EP87/00466**

(87) Internationale Veröffentlichungsnummer :
**WO 88/01162 25.02.88 Gazette 88/05**

(54) **MITTEL ZUM OXIDATIVEN FÄRBEN VON HAAREN, VERFAHREN ZU SEINER HERSTELLUNG UND VERWENDUNG DES MITTELS.**

(30) Priorität : **21.08.86 DE 3628398**

(43) Veröffentlichungstag der Anmeldung :
**21.09.88 Patentblatt 88/38**

(45) Bekanntmachung des Hinweises auf die
Patenterteilung :
**27.11.91 Patentblatt 91/48**

(45) Bekanntmachung des Hinweises auf die
Entscheidung über den Einspruch :
**20.07.94 Patentblatt 94/29**

(84) Benannte Vertragsstaaten :
**FR GB NL**

(56) Entgegenhaltungen :
**EP-A- 0 026 473
CH-A- 347 814
DE-A- 119 187
DE-A- 2 028 818
DE-A- 2 222 001
DE-C- 1 017 749
FR-A- 326 010
FR-A- 650 725
FR-A- 1 045 530
FR-A- 1 121 036
FR-A- 1 439 307
JP-A- 5 372 836
Recherches Nr. 2 (1937), S. 155, Abstract J.
Saphir, Teintures pour cheveux, Demande All.
S. 119-187
Ullmanns Enzyklopädie der technischen Chemie, 3. Auflage, 10. Band, 1958, S. 741
J. S. Jellinek: Kosmetologie, Dr. Alfred Hüthig
Verlag, Heidelberg, 3. Auflage, 1976, S. 659
K. Schrader, Grundlagen und Rezepturen der
Kosmetika, Dr. Alfred Hüthig Verlag, Heidelberg, 1979, S. 533
K. Venkataraman, The Chemistry of SSynthetic Dyes, Academic Press, New York and London, 1971, Vol V, Kapitel VII "Hair Dyes", S.
475, 479**

(56) Entgegenhaltungen :
**E. Sidi und Ch. Zviak, Problèmes Capillaires,
1966, S. 141. 146
G. T. Walker, 1957, SÖFW 23, 699 - 702
J.S. Jellinek: Kosmetologie, Dr. Alfred Hüthig
Verlag, Heidelberg, 3. Auflage, 1976, S. 660
J.C. Johnson "Hair Dyes", Noyes Data Corporation (1973), Seiten 94 - 111 und 250 - 255**

(73) Patentinhaber : **GOLDWELL
AKTIENGESELLSCHAFT
Zerninstrasse 10-18
D-64297 Darmstadt (DE)**

(72) Erfinder : **TENNIGKEIT, Jürgen
Felsbergstrasse 51
D-6104 Seeheim 2 (DE)**
Erfinder : **LORENZ, Herbert
Ober Ramstädter Strasse 22
D-6101 Gross-Bieberau (DE)**

## Beschreibung

Die Erfindung betrifft ein Mittel für die oxidative Färbung von menschlichen und tierischen Haaren, welches unmittelbar vor der Anwendung durch Vermischung eines wenigstens einen Oxidationsfarbstoff enthaltenden creme- oder gelförmigen Färbemittels mit einem Oxidationsmittel hergestellt ist, einen Katalysator enthält und auf einen pH-Wert zwischen 5,9 und 6,9 eingestellt ist. Außerdem betrifft die Erfindung ein Verfahren zur Herstellung des Mittels sowie seine Verwendung.

Bis in die jüngste Zeit werden für die dauerhafte Färbung von menschlichen Haaren alkalische Oxidationsfarbstoffe enthaltende Färbemittel in Gel- oder Cremeform verwendet, die unmittelbar vor der Anwendung mit einem sauren Oxidationsmittel, z.B. Wasserstoffperoxid, zum gebrauchsfertigen auf das zu färbende Haar aufzutragende Haarfärbemittel vermischt werden. Die Färbung entsteht hierbei durch Reaktion bestimmter Entwicklersubstanzen mit bestimmten Kupplersubstanzen in Gegenwart eines geeigneten Oxidationsmittels. Das gebrauchsfertige Präparat liegt dabei deutlich im alkalischen Bereich, wodurch die Oberflächen der zu färbenden Haare in eine für das Eindringen der Oxidationsfarbstoffe vorteilhaften Weise aufgeschlossen werden. Andererseits beanspruchen alkalische Präparate die Haare auch, so daß es - insbesondere bei wiederholten Einwirkungen, z.B. bei mehrfachem Nachfärben - zu Schädigungen des Haars kommen kann. Solche Nachfärbungen sind aber- insbesondere bei heller gefärbtem Haar- erforderlich, um beispielsweise den ungefärbten Haamachwuchs farblich an die bereits gefärbten Haarlängen anzugleichen, bzw. um die früher gefärbten Haarlängen farblich aufzufrischen, wenn sie im Laufe der Zeit durch äußere Einwirkungen, z.B. Sonnenbestrahlung, häufiges Waschen u.dgl. gegenüber der ursprünglichen Farbe aufgehellt bzw. ausgeblichen sind.

Eine Möglichkeit, die Beanspruchung der Haare durch den Färbevorgang zu vermindem, bestünde darin, daß man die Einwirkzeit des alkalischen Präparats herabsetzt - beispielsweise durch Zugabe geeigneter Katalysatoren. So ist es aus der FR-A-1439307 bekannt, oxidativen Haarfärbemitteln mit pH-Werten zwischen 5 und 10 zur Beschleunigung des Oxidationsvorgangs Chelatkomplexe beizugeben, die Metalle der Gruppe Cr. Mn, Fe, Co, Ni und Cu als Katalysatoren enthalten. Für die gebrauchsfertige, auf das Haar aufzutragende Haarfärbemischung kann so die Einwirkzeit bei alkalischer Einstellung des Mittels auf nur 10 Minuten gegenüber sonst 30 Minuten begrenzt werden. Die strukturschädigende Wirkung des alkalischen Präparates wird dadurch jedoch noch nicht verhindert oder im gewünschten Maß vermindert.

Frühere Versuche, die insbesondere in den Haarspitzen auftretenden schädigenden Wirkungen der alkalischen Haarfärbemittel durch saure Einstellung des aus dem Oxidationsfarbstoff und dem Oxidationsmittel fertig aufbereiteten Präparats zu vermeiden, führten zu einer ungenügenden Anfärbewirkung des Mittels. Erst in jüngster Zeit ist von der Anmelderin ein im gebrauchsfertig aufbereiteten Zustand im schwach saurem Bereich (pH-Wert zwischen 5,9 und 6,9) liegendes Mittel entwickelt worden, welches eine gute Anfärbung der behandelten Haare bewirkt, wobei die bei alkalischen Haarfärbemitteln zu beobachtenden Schädigungen der Haare weitestgehend vermieden sind (W0 87/01033) Erreicht wird dieses überraschende Ergebnis bei diesem Haarfärbemittel durch Zugabe von Mangandioxid zum Haarfärbemittel in geringen Mengen, welches bezüglich der Anfärbewirkung offensichtlich als Katalysator wirkt.

Der Erfindung liegt die Aufgabe zugrunde, ein Mittel zum oxidativen Färben von Haaren sowie ein Verfahren zu seiner Herstellung anzugeben, welches bei noch verbesserter Färbewirkung auch bei mehrfacher Anwendung nicht zu den bei Verwendung von alkalischen Haarfärbemitteln beobachteten Haarschädigungen führt.

Ausgehend von einem Haarfärbemittel der eingangs erwähnten Art wird diese Aufgabe erfindungsgemäß dadurch gelöst, daß es auf einen pH-Wert zwischen 5,9 und 6,9 eingestellt ist und daß es als Katalysatorwenigstens ein Metallsalz aus der Gruppe Kupfer-II-chlorid, Kupfer-II-sulfat, Kobaltchlorid, Cersulfat und Vanadiumsulfat enthält.

Dabei hat sich gezeigt, daß das Metallsalz im behandlungsfertigen Haarfärbemittel bereits zu der gewünschten katalytischen Verstärkung der Anfärbewirkung führt, wenn es in Anteilen zwischen 0,0001 und 1,00 Gew.%, vorzugsweise zwischen 0,0004 und 0,001 Gew.% enthalten ist. Im Vergleich zu dem obenerwähnten neu entwickelten, mit Mangandioxid-Pulver als katalytischen Zusatz wirkenden sauer eingestellten Haarfärbemittel genügt also beim erfindungsgemäßen Haarfärbemittel ein um eine bis zwei Zehnerpotenzen geringerer Anteil der zuzufügenden Metallsalze.

Bei der Herstellung des erfindungsgemäßen Haarfärbemittels wird entweder so verfahren, daß das Metallsalz dem Oxidationsfarbstoff vor der Zugabe des - in der Regel flüssigen - Oxidationsmittels zugemiscnt wird, oder es kann auch mit oder unmittelbar nach der Vermischung des Oxidationsfarbstoffs mit dem Oxidationsmittel zugegene werden.

Bei der Anwendung des erfindungsgemäßen Haarfärbemittels zum Färben menschlicher Haare wird praktisch in gleicher Weise wie bisher mit alkalischen Haarfärbemitteln gearbeitet. Unmittelbar vor der Anwendung wird aus wenigstens einem creme- oder gelförmigen, einen oder mehrere Oxidationsfarbstoff(e) und zusätzlich das Metallsalz enthaltenden Färbemittel durch Zugabe des Oxidationsmittels das gebrauchsfertige Haarfärbemittel aufbereitet, auf das zu färbende Haar aufgetragen und eine vorgegebene Zeitdauer zur Einwirkung gebracht, worauf das Haarfärbemittel ausgewaschen wird. Es hat sich gezeigt, daß hierbei hervorragend haltbare Färbungen von menschliche Haar in brillänten Farbtönen ermöglicht werden, sofern nicht dunkleres Haar auf einen helleren Farbton umgefärbt werden soll. Die auf die alkalische Einstellung der bis heute überwizgend verwendeten Haarfärbemittel zurückzuführenden Haarschädigungen werden dabei vollständig vermieden.

Für die aufhellende oder heller ziehende Färbung von dunklerem Haar muß allerdings auf die bekannten alkalischen Haarfärbemittel zurückgegriffen werden. Da die Schädigungen der Haare durch solche alkalischen Haarfärbemittel aber in der Regel erst bei mehrfachem Nachfärben auftreten, können solche Schäden beim Nachfärben vermieden werden, indem zunächst auf die dunkleren nachgewachsenen kopfhautnahen Haarlängen ein an sich bekanntes alkalisch eingestelltes Haarfärbemittel und dann das schwach saure Haarfärbemittel auf die bereits früher gefärbten anschließenden Haarlängen aufgebracht wird, worauf sowohl das alkalische als auch das schwach saure Haarfärbemittel nach hinreichender Einwirkungszeit aus dem behandelten Haar ausgewaschen werden.

Besonders problematisch in bezug auf Haarschädigungen ist eine kombinierte Dauerwell-Haarfärbebehandlung. Wesentlich verringert wird die Gefahr von Haarschäden bei einer Anwendung des erfindungsgemäßen Haarfärbemittels, wobei dann so vorgegangen wird, daß auf das gewaschene und auf Wicklem aufgewickelte Haar zunächst ein Dauerwellpräparat für eine vorgegebene Zeitdauer zur Einwirkung gebracht und dann ausgewaschen wird, worauf das gewickelte Haar mit einem flüssigen oxidierenden Fixiermittel vorfixiert und dann nach dem Abnehmen der Wickler mittels des schwach sauren Haarfärbemittels nachfixiert wird. indem dieses für die zur hinreichenden Einfärbung der Haare erforderliche Zeitdauer auf dem Haar zur Einwirkung gebracht und anschließende ausgewaschen wird. Das Haarfärbemittel ersetzt also das beim Dauerwellen üblicherweise beim Nachfixieren verwendete Fixiermittel, d.h. wird sozusagen in Doppelfunktion eingesetzt, wobei die sonst bei Verwendung von alkalisch eingestellten Haarfärbemitteln zu befürchtenden Schädigungen vermieden werden.

Die vorstehend geschilderten Verwendungsmöglichkeiten des erfindungsgemäßen Haarfärbemittels werden nachstehend anhand einer Reihe von speziellen Beispielen näher ausgeführt. Als Beispiel für die Wirksamkeit von Metallsalzen werden dabei Rezepturen von unter Zugabe von Kupfer-II-chlorid aufbereiteten Haarfärbemitteln beschrieben.

Beispiel 1a

Oxidationsfärbung

20 ml Färbepaste der unten angegebenen Rezeptur für die Farbe "Haselnußblond" mit einem Zusatz von 0,0004 % Kupfer-II-chlorid wurde mit 40 ml einer 2 % $H_2O_2$ enthaltenden Entwicklerlösung vermischt, woraus ein Haarfärbemittel mit einem pH-Wert von 6,8 resultierte. Das Mittel wurde auf ein mittelstarkes Haar mit dem Grundton mittelaschblond aufgetragen und 20 Minuten zur Einwirkung gebracht.

Als Ergebnis wurde eine Umfärbung des Haares zu einem intensiven Haselnußblond-Farbton erhalten.

Versuche ergaben, daß die Einwirkungszeit des Haarfärbemittels je nach gewünschter Farbintensität 5 bis 20 Minuten betragen kann.

Rezeptur der Färbepaste "Haselnußblond":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,25 g |
| m-Aminophenol | 0,01 g |
| Resorcin | 0,01 g |
| p-Aminophenol | 0,08 g |
| p-Amino-o-kresol | 0,06 g |
| Pikraminsäure | 0,05 g |
| Monoäthanolamin | 0,2 g |
| Ammoniumchlorid | 0,2 g |
| Natriumlaurylsulfat | 0,3 g |
| Kupfer-II-chlorid | 0,0004 g |
| Natriumsulfit | 0,25 g |
| Äthylendiamintetraessigsäure | 0,2 g |
| Parfum | 0,2 g |
| Enth. Wasser auf | 100,00 g |

Beispiel 1b

Oxidationsfärbung

20 ml Färbepaste der unten angegebenen Rezeptur für die Farbe "Kupferblond" mit einem Zusatz von 0,0004 % Kupfer-II-chlorid wurde mit 40 ml einer 2 % $H_2O_2$ enthaltenden Entwicklerlösung vermischt, woraus ein Haarfärbemittel mit einem pH-Wert von 6,8 resultierte.

Das Mittel wurde auf ein mittelstarkes Haar mit dem Grundton mittelaschblond augetragen und 20 Minuten zur Einwirkung gebracht. Als Ergebnis wurde eine Umfärbung des Haars in einen dezenten Kupferton erhalten.

Die Einwirkungszeit des Haarfärbemittels kann je nach gewünschter resultierender Farbintensität 5 bis 30 Minuten betragen.

Rezeptur der Färbepaste "Kupferblond":

| | |
|---|---:|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,20 g |
| p-Aminophenol | 0,70 g |
| p-Amino-o-kresol | 0,70 g |
| Monoäthynolamin | 0,2 g |
| Ammoniumchlorid | 0,2 g |
| Natriumlaurylsulfat | 0,3 g |
| Kupfer-II-chlorid | 0,0004 g |
| Natriumsulfit | 0,12 g |
| Äthylendiamintetraessigsäure | 0,2 g |
| Parfum | 0,2 g |
| Enth. Wasser | auf 100,00 g |

Beispiel 2

Oxidationsfärbung zum schonenden, sauren Ausgleich der Haarspitzen nach normaler, alkalischer Ansatz-färbung

20 ml Färbepaste der beim Beispiel 1a angegebene Rezeptur für die Färbe "Haselnußblond" mit einem Zusatz von 0,0004 % Kupfer-II-chlorid wurde mit 40 ml einer 2 % $H_2O_2$ enthaltenden Entwicklerlösung vermischt, woraus ein Haarfarbemittel mit einem pH-Wert von 6,8 resultierte.

Das Mittel wurde auf ein mittelstarkes Haar, welches haselnußblond gefärbt war und dessen Längen und Spitzen ausgeblichen sind und einen Ton von hellaschblond haben, aufgetragen und 10 Minuten zur Einwirkung gebracht.

Als Ergebenis wurde ein dem Ansatz angeglichener Haselnußblond-Farbton der ausgeblichenen Längen und Spitzen erhalten. Die Einwirkungsdauer des Präparats kann wiederum entsprechend den auszugleichenden Farbintensitäten zwischen 5 bis 15 Minuten variiert werden.

Beispiel 3a

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Zyklamen" mit einem Zusatz von 0,0004 % Kupfer-II-chlorid mit 40 ml einer Dauerwell-Fixierung mit einem $H_2O_2$-Gehalt von 2 % vermischt.

Das hieraus resultierende Haarfärbemittel mit einem pH-Wert von 6,8 wurde nun mittels einer Auftrageflasche bzw. eines Scwämmchens auf das Haar aufgetragen und 10 Minuten zur Einwirkung gebracht. Als Ergebnis wurde zusätzlich zur Dauerwellung eine Umfärbung des Haars in einen rotvioletten (Zyklamen)-Farbton erhalten. Je nach gewünschter Farbinten sität kann die Einwirkungsdauer zwischen 5 und 15 Minuten variiert werden.

5

Rezeptur der Färbepaste "Zyklamen":

| | |
|---|---|
| Cetylstearylalkohol | 10,0 g |
| Kokosfettsäuremonoäthanolamid | 2,0g |
| Stearinsäuremonoäthanalamid | 2,0 g |
| Stearinsäurediäthanolamid | 1,0 g |
| p-Toluylendiaminsulfat | 0,5 g |
| p-Amino-o-kresol | 0,4 g |
| p-Aminophenol | 0,1 g |
| Monoäthanolamin | 0,2 g |
| Ammoniumchlorid | 0,2 g |
| Natriumlaurylsulfat | 0,3 g |
| Kupfer-II-chlorid | 0,0004 g |
| Natriumsulfit | 0,25 g |
| Äthylendiamintetraessigsäure | 0,2 g |
| Parfum | 0,2 g |
| Enth. Wasser | auf 100,00 g |

Beisqiel 3b

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Mahagoni" mit einem Zusatz von 0,0004 Kupfer-II-chlorid mit 40 ml einer Dauerwell-Fixierung mit einem $H_2O_2$-Gehalt von 2 % vermischt.

Das hieraus resultierende Haarfärbemittel mit einem pH-Wert von 6,8 wurde nun mittels einer Auftrageflasche bzw. eines Schwämmchens auf das Haar aufgetragen und 10 Minuten zur Einwirkung gebracht Als Ergebnis wurde zusätzlich zur Dauerwellung eine Umfärbung des Haars in einen kräftigen Rotton erhalten. Je nach gewünschter Farbintensität kann die Einwirkungdauer zwischen 5 und 15 Minuten variiert werden.

## Rezeptur der Färbepaste "Mahagoni":

| | |
|---|---|
| Cetylstearylalkohol | 10,0 g |
| Kokosfettsäuremonoäthanolamid | 2,0 g |
| Stearinsäuremonoäthanolamid | 2,0 g |
| Stearinsäurediäthanolamid | 1,0 g |
| p-Toluylendiaminsulfat | 0,4 g |
| p-Aminophenol | 0,4 g |
| p-Amino-o-kresol | 0,4 g |
| o-Nitro-p-Phenylendiamin | 0,4 g |
| Monoäthanolamin | 0,2 g |
| Ammoniumchlorid | 0,2 g |
| Natriumlaurylsulfat | 0,3 g |
| Kupfer-II-chlorid | 0,0004 g |
| Natriumsulfit | 0,25 g |
| Äthylendiamintetraessigsäure | 0,2 g |
| Parfum | 0,2 g |
| Enth. Wasser | auf 100,00 g |

Versuche haben gezeigt, daß neben dem in den Beispielen 1a bis 3a verwendeten Kupfer-II-chlorid auch die Metallsalze Kupfer-II-sulfat, Kobaltchlorid, Cersulfat und Vanadiumsulfat als Zuschlagstoffe zu Haarfärbemitteln die Wirkung haben, auch bei sauer eingestellter und somit das zu behandelnde Haar schonender Rezeptur eine brillante und farbintensive Umfärbung oder Farbauffrischung des behandelten Haars zu ermöglichen.

**Patentansprüche**

1. Mittel für die oxidative Färbung von menschlichen oder tierischen Haaren, welches unmittelbar vor der Anwendung durch Vermischen eines wenigstens einen aus Entwickler- und Kupplersubstanzen entstehenden Oxidationsfarbstoff enthaltenden creme- oder gelförmigen Färbemittels mit einem Oxidationsmittel hergestellt ist, einen Katalysator enthält und einen pH-Wert zwischen 5 und 10 hat, **dadurch gekennzeichnet,** daß es auf einen pH-Wert zwischen 5,9 und 6,9 eingestellt ist und daß es als Katalysator wenigstens ein Metallsalz aus der Gruppe Kupfer(II)chlorid, Kupfer(II)sulfat, Kobaltchlorid, Cersulfat und Vanadiumsulfat enthält.

2. Haarfärbemittel nach Anspruch 1, dadurch gezennzeichnet, daß das Metallsalz im behandlungsfertigen Haarfärbemittel in Anteilen zwischen 0,0001 und 1,00 Gew.%, vorzugsweise zwischen 0,0004 und 0,001 Gew.% enthalten ist.

3. Verfahren zur Herstellung des Haarfärbemittels nach Ansprüch 1 oder 2, dadurch gekennzeichnet, daß das Metallsalz dem Oxidationsfarbstoff vor der Zugabe des Oxidationsmittels zugemischt wird.

4. Verfahren zur Herstellung des Haarfärbemittels nach Ansprüch 1 oder 2, dadurch gekennzeichnet, daß das Metallsalz bei der Aufbereitung des Haarfärbemittels zugleich mit oder unmittelbar nach der Vermischung des Oxidationsfarbstoffs mit dem Oxidationsmittel zugegeben wird.

5. Verfahren nach Anspruch 3 oder 4, dadurch gezennzeichnet, daß dem Haarfärbemittel Metallsalz in solcher Menge zugegeben wird, daß es im gebrauchsfertigen Haarfärbemittel mit einem Anteil zwischen 0,0001 und 1,00 Gew.%, vorzugsweise zwischen 0,0004 und 0,001 Gew.% enthalten ist.

6. Verwendung des Haarfärbemittels nach Anspruch 1 oder 2 zum oxidativen Färben von menschlichen oder tierischen Haaren welches unmittelbar vor der Anwendung aus wenigstens einem aus Entwickler- und Kupplersubstanzen entstehenden Oxidationsfarbstoff und einem Oxidationsmittels zum gebrauchtfähigen Haarfärbemittel aufbereitet, auf das zu färbende Haar aufgetragen und eine vorgegebene Zeitdauer zur Einwirkung gebracht wird, worauf das Haarfärbemittel ausgewaschen wird.

7. Verwendung des Haarfärbemittels nach Anspruch 1 oder 2 zum Nachfärben von im Naturzustand dunklerem heller gefärbtem Haar, wobei zunächst auf die dunkleren, nachgewaschenen hautnahen Haarlängen ein an sich bekanntes alkalisch eingestelltes Haarfärbemittel und dann das schwach Haarfärbemittel auf die bereits früher gefärbten anschließenden Haarlängen aufgebracht wird, worauf sowohl das alkalische als auch das schwach saure Haarfärbemittel nach hinreichender Einwirkungszeit aus dem behandelten Haar ausgewaschen werden.

8. Verwendung des Haarfärbemittels nach Anspruch 1 oder 2 beim Wellen und oxidativen Färben von Haaren, wobei auf das gewaschene und auf Wicklern aufgewickelte Haar zunächst ein Dauerwellpräparat für eine vorgegebene Zeitdauer zur Einwirkung gebracht und dann ausgewaschen wird, worauf das gewickelte Haar mit einem flüssigen oxidierenden Fixiermittel vorfixiert und dann nach dem Abnehmen der Wickler mittels des schwach sauren Haarfärbemittels nachfixiert wird, indem dieses für die zur hinreichenden Einfärbung der Haare erforderliche Zeitdauer auf dem Haar zur Einwirkung gebracht und anschließend ausgewaschen wird.

## Claims

1. Agent for the oxidative colouring of human or animal hair, which is prepared immediately before use by mixing a cream-form or gel-form colorant containing at least one oxidative dye being produced from developing and coupling substances with an oxidising agent, and which contains a catalyst and which has a pH value of between 5 and 10, characterised in that it has been adjusted to a pH value of between 5.9 and 6.9 and it contains as catalyst at least one metal salt from the group copper(II) chloride, copper(II) sulphate, cobalt chloride, cerium sulphate and vanadium sulphate.

2. Hair colorant according to claim 1, characterised in that the metal salt is present in the ready-to-use hair colorant in proportions of between 0.0001 and 1.00% by weight, preferably between 0.0004 and 0.001% by weight.

3. Process for the preparation of the hair colorant according to claim 1 or 2, characterised in that the metal salt is mixed with the oxidative dye before the addition of the oxidising agent.

4. Process for the preparation of the hair colorant according to claim 1 or 2, characterised in that during the preparation of the hair colorant the metal salt is added at the same time as or immediately after the mixing of the oxidative dye with the oxidising agent.

5. Process according to claim 3 or 4, characterised in that the metal salt is added to the hair colorant in such an amount that it is present in the ready-to-use hair colorant in a proportion of between 0.0001 and 1.00% by weight, preferably between 0.0004 and 0.001% by weight.

6. The use of the hair colorant according to claim 1 or 2 for the oxidative colouring of human or animal hair, which is prepared immediately before use from at least one oxidative dye being produced from developing and coupling substances and an oxidising agent to form the usable hair colorant and is then applied to the hair to be coloured and allowed to act for a predetermined period of time, after which the hair colorant is washed out.

7. The use of the hair colorant according to claim 1 or 2 for the recolouring of naturally relatively dark hair that has been dyed a lighter shade, wherein firstly an alkaline hair colorant known per se is applied to the darker regrowth hair close to the skin and then the weakly acidic hair colorant is applied to the adjoining, previously dyed hair and then, after a sufficient action time has elapsed, both the alkaline and the weakly acidic hair colorants are washed out of the treated hair.

8. The use of the hair colorant according to claim 1 or 2 for the waving and oxidative colouring of hair, wherein firstly a permanent wave preparation is allowed to act for a predetermined time period on the hair, which has been washed and wound in rollers, and is then washed out, and then the hair wound in rollers is given preliminary fixing with a liquid oxidising fixing agent and then, after the rollers have been removed, subjected to after-fixing using the weakly acidic hair colorant by allowing the colorant to act on the hair for the time period necessary for adequate colouring of the hair and then washing it out.

**Revendications**

1. Agent ou produit pour la teinture par oxydation de cheveux humains ou de poils d'animaux, produit que l'on prépare immédiatement avant l'application par mélange d'une matière colorante en forme de crème ou de gel contenant au moins un colorant par oxydation se formant d'agents de développement et couplage avec un oxydant, ce produit contenant un catalyseur et ayant un pH compris entre 5 et 10, produit caractérisé en ce qu'on l'ajuste à un pH compris entre 5,9 et 6,9 et en ce qu'il contient comme catalyseur au moins un sel de métal choisi dans l'ensemble constitué par le chlorure de cuivre-(II), le sulfate de cuivre-(II), le chlorure de cobalt, le sulfate de cérium et le sulfate de vanadium.

2. Produit pour la teinture des cheveux selon la revendication 1, caractérisé en ce que le sel de métal est contenu, dans le produit prêt à servir pour la teinture des cheveux, en des proportions comprises entre 0,0001 et 1,00 % en poids, avantageusement entre 0,0004 et 0,001 % en poids.

3. Procédé pour prépararer le produit ou l'agent pour teinture des cheveux selon les revendications 1 ou 2, caractérisé en ce qu'on incorpore le sel de métal au colorant à obtenir par oxydation, avant d'ajouter l'oxydant.

4. Procédé pour préparer le produit pour teinture de cheveux selon les revendications 1 ou 2, caractérisé en ce qu'on ajoute le sel de métal, lors de la préparation du produit pour teinture de cheveux, en même temps ou immédiatement après le mélange du colorant d'oxydation avec l'oxydant.

5. Procédé selon la revendication 3 ou 4, caractérisé en ce qu'on ajoute au produit pour la teinture des cheveux le sel de métal en une quantité telle que le sel soit présent, dans le produit prêt à servir à la teinture des cheveux, en une proportion comprise entre 0,0001 % et 1,00 % en poids, avantageusement entre 0,0004 et 0,001 % en poids.

6. Utilisation de l'agent ou produit pour teinture de cheveux selon la revendication 1 ou 2 pour la teinture oxydante de cheveux humains ou de poils d'animaux, que l'on prépare immédiatement avant l'application à partir d'au moins un colorant d'oxydation se formant d'agents de développement et couplage et d'un oxydant pour obtenir un produit, prêt à servir à la teinture des cheveux, que l'on applique sur la chevelure à teindre et qu'on laisse agir pendant une période de temps prédéterminée, après quoi on élimine par lavage le produit pour la teinture des cheveux.

7. Utilisation du produit pour teinture des cheveux selon les revendication 1 ou 2 pour teindre après coup une chevelure qui a été teinte en une nuance plus claire que son état naturel plus foncé, selon laquelle on applique tout d'abord, sur les longueurs de cheveux proches du cuir chevelu, qui sont plus foncés et ont été soumises à un post-lavage, un produit connu en soi, ajusté à un pH alcalin, pour teinture des cheveux et l'on applique ensuite le produit faiblement acide pour teinture de cheveux sur les longueurs de cheveux déjà préalablement teintes, puis l'on élimine par lavage, de la chevelure ainsi traitée, aussi bien le produit alcalin que le produit faiblement acide pour teinture de cheveux, après les avoir laissés agir pendant un temps suffisant.

8. Utilisation du produit pour teinture des cheveux selon la revendication 1 ou 2 dans le cas d'une ondulation et d'une teinture par oxydation des cheveux, selon laquelle on fait agir tout d'abord sur les cheveux lavés et enroulés sur des bigoudis une préparation pour permanente, qu'on laisse agir pendant une période de temps prédéterminée puis qu'on élimine par lavage, après quoi on soumet à préfixage, à l'aide d'un produit liquide oxydant pour fixation, les cheveux enroulés puis, après enlèvement des bigoudis, on fixe ensuite à l'aide du produit faiblement acide pour teinture de cheveux, en laissant agir ce dernier produit sur la chevelure pendant la période de temps nécessaire pour une pénétration suffisante de la nucance de teinte dans les cheveux, puis on élimine les produits par lavage.